# EUROPEAN PATENT SPECIFICATION

(11) **EP 1 384 489 B1**
(45) Date of publication and mention of the grant of the patent: **26.03.2008**
(21) Application number: 02077989.8
(22) Date of filing: 22.07.2002
(51) Int. Cl.: A61L 27/44, A61L 27/58, A61F 2/10, A61F 2/30

(54) **Biocompatible scaffold as a dermal or cartilage substitute**
Biokompatibles Gerüst als Haut- oder Knorpelersatz
Greffe biocompatible comme substitut de peau ou de cartilage

(43) Date of publication of application: 28.01.2004
(73) Proprietor: OctoPlus Sciences B.V., 2333 CL Leiden (NL)
(72) Inventor: Pieper, Jeroen, 3720 AB Bilthoven (NL); Lamme, Evert Nicolaas, 3720 AB Bilthoven (NL)
(74) Representative: Prins, Adrianus Willem

(56) References cited:
- US-B1- 6 383 220
- VAN DORP A G ET AL: "Dermal regeneration in full-thickness wounds in Yucatan miniature pigs using a biodegradable copolymer." WOUND REPAIR AND REGENERATION: OFFICIAL PUBLICATION OF THE WOUND HEALING SOCIETY [AND] THE EUROPEAN TISSUE REPAIR SOCIETY. UNITED STATES 1998 NOV-DEC, vol. 6, no. 6, November 1998 (1998-11), pages 556-568, XP001118066 ISSN: 1067-1927
- VAN DORP ANNETTE G M ET AL: "Bilayered biodegradable poly(ethylene glycol)/poly(butylene terephthalate) copolymer (Polyactive(R)) as substrate for human fibroblasts and keratinocytes." JOURNAL OF BIOMEDICAL MATERIALS RESEARCH, vol. 47, no. 3, 5 December 1999 (1999-12-05), pages 292-300, XP001118045 ISSN: 0021-9304
- JUX CHRISTIAN ET AL: "Experimental ASD closure using autologous cell-seeded interventional closure devices." CARDIOVASCULAR RESEARCH. NETHERLANDS JAN 2002, vol. 53, no. 1, January 2002 (2002-01), pages 181-191, XP001118603 ISSN: 0008-6363
- VAN DIJKHUIZEN-RADERSMA R ET AL: "Control of vitamin B12 release from poly(ethylene glycol)/poly(butylene terephthalate) multiblock copolymers" BIOMATERIALS, ELSEVIER SCIENCE PUBLISHERS BV., BARKING, GB, vol. 23, no. 6, 15 March 2002 (2002-03-15), pages 1527-1536, XP004348163 ISSN: 0142-9612

## Description

The present invention relates to a biocompatible scaffold comprising a biocompatible polymer and a protein selected from the group consisting of collagens and biological precursors and functional analogues thereof which is manufactured by the method of claim 1.

Collagen is the main structural protein in men and is frequently used in the biomedical field due to its good biological properties, its availability and its biocompatibility. In addition, collagen is biodegradable, which allows remodelling and replacement with new host tissue upon implantation. Applications of collagen, include its use as a vascular sealant, tissue adhesive and as a temporary scaffold for tissue engineering of *inter alia* skin and cartilage. However, mechanical and handling properties of collagenous scaffolds are generally poor, even after cross-linking of the collagen. Furthermore, despite extensive cross-linking procedures, collagen based scaffolds are rapidly degraded, limiting their use.

In US patent 5,292,802, it has been proposed to coat synthetic tubes with collagen by covalent immobilization of the collagen with difunctionally activated polyethylene glycol.

US 6,071,447 describes the use of polyurethane as a cross-linker to prepare polyurethane-collagen composites for drug delivery purposes. Polyurethane, however, is not or hardly biodegradable and is therefore less useful for *in vivo* applications.

US 5,131,907 describes an intravascular implant, seeded with microvascular endothelial cells, wherein collagen is covalently bound to a polyester substrate, using glutaraldehyde as a cross-linking agent to improve the adherence and proliferation of endothelial cells.

EP 0,784,986 relates to artificial hairs that can be implanted. The hairs are made of a graft-polymerised chain and are chemically coated with a protein, *e.g.* collagen.

It is an object of the present invention to provide a novel biocompatible scaffold that can be used for a medical purpose, in particular as a tissue-substitute or a drug delivery device, which scaffold can be used as an alternative to known biocompatible scaffolds.

It has been found that this object is realised by a biocompatible scaffold comprising a specific biocompatible synthetic polymer and one or more proteins selected from a specific group of proteins manufactured by the method of claim 1.

Accordingly, the present invention relates to a biocompatible scaffold comprising (a) at least one protein selected from the group consisting of collagens, biological precursors of a collagen and collagen-like peptides and (b) a copolymer of a polyalkylene glycol terephtalate and an aromatic polyester.

A scaffold according to the invention has been found very suitable for use as a dermal substitute, as a cartilage-substitute, a vascular substitute or other tissue substitute, as a cell carrier or as a drug-delivery system.

It has been found that a scaffold according to the invention typically has the advantageous mechanical and handling properties of the copolymer. In particular the copolymer of the scaffold gives a high level of flexibility to the scaffold. The hydrophilicity and biodegradability of the copolymer are usually hardly affected or not affected at all, after being provided with the protein.

The scaffold also has the advantages of specific biological signalling sites provided by the protein, which allows the scaffold to actively participate in a healing process after application at the body or implantation into the body.

Further, it has been found that the protein contributes significantly to the suitability of a scaffold according to the invention as an *in vivo* drug delivery system, as the protein allows better loading of the scaffold with one or more pharmaceutically active substances, biopolymers and/or extracellular matrix materials, which can be incorporated into the protein or polymer phase of the scaffold (*e.g.* the coating). The protein can be combined with the copolymer in an efficient and convenient manner, allowing bioactive agents to be incorporated without the necessity to involve drastic measures that could affect the stability or activity of the bioactive agents, such as the use of organic solvents or high temperature. A scaffold according to the invention is moreover easy to handle, sterilise, store and transport.

The term biological precursor of collagen as used herein is used for peptides, polypeptides and proteins that can be transformed into collagen by a biological process, *e.g.* enzymatically. Preferred examples of such precursors are procollagens. Much preferred is a heterotrimer procollagen.

A procollagen is a soluble precursor of fibrillar collagen and is characterised by the presence of globular extension peptides (propeptides) at both the amino (N)- and carboxyl (C)-termini, in addition to the helical domain of the protein. The propeptides confer solubility under physiological conditions, so their removal is normally a prerequisite for the formation of insoluble collagen fibrils. It will be understood that in case a globular extension peptide is present at only one of the amino (N)- and carboxyl (C)-termini, the protein is also referred to as a precursor of collagen.

An advantage of using procollagen is its reduced immunogenicity and higher purity in comparison to collagen, when the collagen has not been treated to remove non-collagenous proteins and the non-helical telopeptides which may be attached to the collagen. Procollagen may also contribute to processes in the body, such as neovascularisation and wound-healing in general.

The term functional analogue of collagen or a biological precursor of collagen is used herein to describe biocompatible polymers of amino acids that have similar structural properties to collagen or a biological precursor of collagen. In particular, the term relates to polymers having a relatively high content of glycine in comparison to globular proteins *e.g.* up to one third of the amino acids. Such peptides further tend to have a high degree of regularity with respect to the distribution of glycine. This amount will typically be less in case prodomains of precursors of collagen are taken into account. Further proline is usually highly abundant. Often such peptides also comprise 4-hydroxyproline and/or 5-hydroxylysine. Typically, glycine, proline and hydroxyproline together make up about two thirds of all amino acids in a collagen or precursor or functional analogue thereof.

The protein in the scaffold can be of any origin. Particularly suitable are proteins of human origin, animal derived (particularly bovine) origin and recombinant proteins. Much preferred are type I, type II and type III collagen and type I, type II and type III procollagen.

Recombinant proteins are particularly preferred. Such a protein can be obtained in a virtually unlimited supply with high yields and high purity. Furthermore, recombinant proteins can be produced free of viruses (*e.g.* HIV or hepatitis virus in protein products of human origin) or prions (*e.g.* prions causing BSE in protein products of bovine or human origin). Suitable methods to prepare recombinant proteins, such as human collagen, are known in the art, *e.g.* from WO 97/14431, US 5,593,859, US 6,111,165 and EP-A 0,9967,226.

The copolymer in a scaffold according to the invention, is a copolymer of a polyalkylene glycol terephtalate and an aromatic polyester. Preferably, the copolymer comprises 20-90 wt.%, more preferably 40-70 wt.% of the polyalkylene glycol terephtalate, and 80-10 wt.%, more preferably 60-30 wt.% of the aromatic polyester. A preferred type of copolymers according to the invention is formed by the group of block copolymers.

The polyalkylene glycol terephtalate may have a weight average molecular weight of about 150 to about 10,000. Preferably, the polyalkylene glycol terephtalate has a weight average molecular weight of 200 to 4,000. The aromatic polyester preferably has a weight average molecular weight of from 200 to 5,000, more preferably from 250 to 4,000. The weight average molecular weight of the copolymer preferably lies between 10,000 and 300,000, more preferably between 40,000 and 120,000.

The weight average molecular weight may suitably be determined by gel permeation chromatography (GPC). This technique, which is known per se, may for instance be performed using chloroform, hexafluoro isopropanol or m-cresol as a solvent and polystyrene as external standard. Alternatively, a measure for the weight average molecular weight may be obtained by using viscometry (see NEN-EN-ISO 1628-1). This technique may for instance be performed at 25°C using chloroform as a solvent. Preferably, the intrinsic viscosity of the copolymer lies between 0.2 and 1.5 dL/g, which corresponds to a weight average molecular weight between 10,000 and 300,000. Likewise, the more preferred ranges for the weight average molecular weight measured by GPC mentioned above can also be expressed in terms of the intrinsic viscosity.

In a preferred embodiment, the polyalkylene glycol terephtalate component has units of the formula -OLO-CO-Q-CO-, wherein O represents oxygen, C represents carbon, L is a divalent organic radical remaining after removal of terminal hydroxyl groups from a poly(oxyalkylene)glycol, and Q is a divalent organic radical.

Preferred polyalkylene glycol terephtalates are chosen from the group of polyethylene glycol terephtalate, polypropylene glycol terephtalate, and polybutylene glycol terephtalate and copolymers thereof, such as poloxamers. A highly preferred polyalkylene glycol terephtalate is polyethylene glycol terephtalate.

The terms alkylene and polyalkylene generally refer to any isomeric structure, i.e. propylene comprises both 1,2-propylene and 1,3-propylene, butylene comprises 1,2-butylene, 1,3-butylene, 2,3-butylene, 1,2-isobutylene, 1,3-isobutylene and 1,4-isobutylene (tetramethylene) and similarly for higher alkylene homologues. The polyalkylene glycol terephtalate component is preferably terminated with a dicarboxylic acid residue -CO-Q-CO-, if necessary to provide a coupling to the polyester component. Group Q may be an aromatic group having the same definition as R, or may be an aliphatic group such as ethylene, propylene, butylene and the like.

The polyester component preferably has units -O-E-O-CO-R-CO-, wherein O represents oxygen, C represents carbon, E is a substituted or unsubstituted alkylene or oxydialkylene radical having from 2 to 8 carbon atoms, and R is a substituted or unsubstituted divalent aromatic radical.

In a preferred embodiment, the polyester is chosen from the group of polyethylene terephthalate, polypropylene terephthalate, and polybutylene terephthalate. A highly preferred polyester is polybutylene terephthalate.

The preparation of the copolymer will now be explained by way of example for a polyethylene glycol terephtalate/polybutylene terephthalate copolymer. Based on this description, the skilled person will be able to prepare any desired copolymer within the above described class. An alternative manner for preparing polyalkylene glycol terephtalate/polyester copolymers is disclosed in US-A-3,908,201.

A polyethylene glycol terephtalate/polybutylene terephthalate copolymer may be synthesized from a mixture of dimethyl terephthalate, butanediol (in excess), polyethylene glycol, an antioxidant and a catalyst. The mixture is placed in a reaction vessel and heated to about 180°C, and methanol is distilled as transesterification proceeds. During the transesterification, the ester bond with methyl is replaced with an ester bond with butylene and/or the polyethyene glycol. After transesterification, the temperature is raised slowly to about 245°C, and a vacuum (finally less than 0.1 mbar) is achieved. The excess butanediol is distilled off and a prepolymer of butanediol terephthalate condenses with the polyethylene glycol to form a polyethylene/polybutylene terephthalate copolymer. A terephthalate moiety connects the polyethylene glycol units to the polybutylene terephthalate units of the copolymer and thus such a copolymer also is sometimes referred to as a polyethylene glycol terephthalate/polybutylene terephthalate copolymer (PEGT/PBT copolymer).

The scaffold can have a more or less closed structure or a open structure with channels, *e.g.* provided by pores or by a laminar structure of the scaffold. Good results have been achieved with a scaffold comprising interconnected pores, oriented fibres, laminar structures or a combination thereof. A porous scaffold can be obtained in any usual way, *e.g.* by salt leaching. Other methods of preparation of the scaffold, such as solvent casting, mold compression, 3D-printing and lyophilization, are also suitable depending on the desired characteristics of the scaffold. In this regards, reference is also made to Van Dorp AGM, Verhoeven MCH, Koerten HK et al. Dermal regeneration in fullthickness wounds in Yucatan miniature pigs using a biodegradable copolymer. Wound Rep Reg 1998;6:556-568, and Woodfield TBF, Bezemer JM, Pieper JS et al. Scaffolds for tissue engineering of cartilage. Accepted for publication in Critical Reviews in Eukaryotic Gene Expression.

Preferably at least the majority of the pores in a porous scaffold has an average diameter in the range of 20-1,000 µm, more preferably in the range of 400 to 600. Typically, the pores are present in the polymeric phase of the scaffold. The protein phase may partially or completely fill, or cover in case of a coating, the open space formed by the pores in the polymer phase. The free volume provided by the pores and/or the channels between laminar structures is preferably in the range of 50-90%, preferably 70-80%.

In case the scaffold has an open structure, cells can be seeded into the scaffold before application *in vivo* or cells can migrate into the scaffold after being placed in or at the body. A scaffold according to the invention has for example been found very suitable to be seeded with skin cells, such as (dermal) fibroblasts or keratinocytes, cartilage cells, such as chondrocytes, and/or bone cells or bone forming cells, such as bone marrow cells or mesenchymal stem cells. Such scaffolds are for example very suitable for use as skin-substitutes respectively as cartilage substitutes. Accordingly, the invention also encompasses a scaffold as described herein comprising living cells. Preferably the cells are autologous cells, indicating that they originate from the same organism as for which the tissue substitute is intended.

Depending upon its intended use, the at least one protein may be present at at least part of the exterior of the scaffold or throughout at least part of the scaffold. For instance, when intended to be used as a cell carrier or epidermis substitute, it may be advantageous that the protein phase is present as an external coating on the polymer phase. For use as a drug delivery system, it could be useful to have a distribution of the protein phase throughout the polymer phase.

A coating is preferably non-covalently conjugated to the copolymer, more preferably substantially only by molecular forces such as hydrogen bonds and van der Waals forces. Even more preferably, such a scaffold is provided with a coating that adheres to the matrix without a chemical treatment. An advantage of such a scaffold is the high level of nativity and/or a favourable biodegradability of the protein or proteins in the coating. The protein, *e.g.* collagen, thus has been found to retain *inter alia* a high activity as signalling site to cells that attach to the scaffold.

The thickness of the coating preferably is chosen in the range of from 2 to 30 µm.

Scaffolds coated with collagen or a precursor or functional analogue thereof are particularly useful as a cell carrier to improve wound healing. In such applications, cells are transferred from the scaffold to the wound thereby stimulating tissue repair and regeneration. Keratinocytes, for example, can be used to promote healing of partial thickness burns and chronic wounds. Briefly, keratinocytes are seeded on collagen coated scaffolds. Cell seeded scaffolds are subsequently applied to the wounds in an upside-down fashion, i.e. the cell seeded side of the scaffold being in direct contact with the wound. This allows the transfer of undifferentiated keratinocytes which are able to proliferate and actively participate in the healing process. In addition, the use of pre-confluent cells prevents long-term culturing procedures, hence substantially reducing the time required for the delivery of tissue engineered products to the operation theatre.

For instance for drug delivery purposes, the protein phase may be allowed to infiltrate into (*e.g.* into the pores of) the polymer phase thereby producing a composite scaffold material. In accordance with this embodiment, a biologically active substance or agent may be present in the polymer phase or, more preferably, in the protein phase.

In the claimed method, a dispersion of a collagen or precursor or functional analogue thereof is mixed with a copolymer of polyalkylene glycol terephthalate and an aromatic polyester, poured into a mould and lyophilized to form a biocompatible composite scaffold. The copolymer can be applied to the collagen dispersion in any suitable solid physical form like fibers, flakes, films, particles, granules, and porous structures. Preferably, the copolymer is added in the form of porous or nonporous microspheres. The diameter of the microspheres preferably ranges from 10 to 400 µm. In particular applications, the microspheres can be loaded with bioactive agents prior to their incorporation in the biocompatible composite scaffold. In this way, tailored drug delivery vehicles can be created.

The protein is typically present in the range of 0.1 to 15 wt.%, preferably of 2 to 8 wt.%, based on the weight of the biocompatible scaffold.

The scaffold, and in particular the protein phase of the scaffold, may suitably be provided with one or more pharmaceutically active substances. Such agents could be present in a scaffold for use as a tissue substitute or in a scaffold that is specifically intended as a drug delivery device. A scaffold according to the invention has been found particularly suitable to carry at least one pharmaceutically active substance selected from the group consisting of hormones, such as growth factors, such as fibroblast growth factor (FGF), vascular endothelial growth factor (VEGF), insulin-like growth factor (IGF), bone morphogenetic protein (BMP), tissue growth factor (TGF), or platelet-derived growth factors, antibiotics, antimicrobial peptides, anti-inflammatory agents, chemotactic agents, and anti-fungal agents.

The concentration of pharmaceutically active substance can be widely varied, depending upon its intended purpose and the concentration required to provide a pharmaceutically active amount of the substance. The active substance can be present in the protein phase (*e.g.* the protein coating), in the copolymer or in both. Suitable design parameters for a drug delivery device according to the invention wherein the active substance is present in the polymer phase are for example known from US 5,980,948.

In principle, the scaffold can be prepared by any means known in the art, *e.g.* by chemically bonding the protein and the copolymer. It has however been found that prior art materials, which are coated by chemical conjugation such as covalent conjugation of collagen and similar proteins to synthetic polymers, tend to have relatively poor structural characteristics, biodegradability and/or nativity. The presence of native collagen is a pivotal requisite for exerting various biological properties, like cell binding. Biodegradability is important for temporal implants, so that they do not have to be removed surgically, and for drug delivery devices, since the drug-release is *inter alia* controlled via the degradation of the scaffold.

It has been found that by preparing the scaffold in a specific way it possible to obtain a scaffold wherein the collagen, biological precursor of a collagen respectively the collagen-like peptide retain a high level of nativity and/or a highly favourable level of biodegradability.

Accordingly the present invention relates to a method for manufacturing a biocompatible scaffold, wherein a matrix comprising a copolymer of a polyalkylene glycol terephthalate and an aromatic polyester aromatic polyester is provided; the matrix is contacted with a homogeneous collagen dispersion (in case of a non-soluble collagen) or an aqueous solution (in case of a soluble collagen) comprising at least one protein selected from the group consisting of collagens, biological precursors of a collagen and collagen-like peptides; and wherein the matrix is subsequently dried by lyophilisation to form the scaffold. The scaffold may be subjected to sterilization or any other treatments, such as a gas plasma treatment to enhance cell adhesion.

The method has been found very suitable to provide a scaffold with a coating comprising one or more of said proteins. The method has been found to be highly efficient, particularly for hydrophilic scaffolds, reducing the amount of collagen necessary. The method is very suitable to manufacture the scaffolds reproducibly with high purity in various geometrical shapes.

The mixture comprising the at least one protein is preferably a solution or dispersion of the at least one protein in water or an aqueous solution. The mixture is may be acidic, depending on the initial collagen structure. In case a water insoluble collagen is used, the mixture preferably has a pH in the range of 1-3. In case a water soluble collagen is used, the mixture preferably has a pH in the range 4-8.

As a buffer to reach the suitable pH any physiologically acceptable buffer may be used. Good results have been achieved with a buffer comprising acetic acid. The concentration of protein or proteins in the mixture is preferably chosen in the range of 0.1 to 8% (total weight of proteins/volume of mixture) more preferably in the range of 0.25 to 2% (total weight of proteins/volume of mixture). If the scaffold is to contain one or more pharmaceutically active substances or physiological substances, such as extracellular matrix constituents, these can be added to the mixture in the desired concentration.

The contacting of the matrix and the mixture comprising the at least one protein can for example be done by spraying or submerging (depending on the viscosity of the mixture) the matrix with or in the mixture or submerging the matrix fully or partially in the mixture. The contacting is preferably performed at a temperature of 4 to 37°C. A suitable duration can routinely be determined. Usually a duration of 2 to 4 hours will suffice.

It has further been found that the structure of the phase of the scaffold containing the at least on protein (such as the coating) can be modulated by either letting the mixture and the matrix contact another without any relative movement of both phases, by shaking a reactor containing matrix and mixture or by applying a centrifugal force. For example, it has been found that a matrix coated with a collagen while shaking during the contacting results in a complete coverage of the matrix with a collagenous meshwork comprising a fibrous, porous morphology. By centrifuging matrix while in contact with a mixture comprising *e.g.* collagen the formation of sheet-like coating structures can be favoured. Preferred centrifugation conditions are 200-1000 xg, 5-30 min, 4-20 °C. The presence of sheet-like structures may be useful to prevent the ingrowth of cells in the porous synthetic scaffold (synthetic scaffold is coated with sheet-like layer). For example for the creation of an epidermis, or for the cell carrier system (presence of cells only at the outer layer of the biocompatible scaffold may result in more efficient cell transfer to wounds).

The claimed method leads to a coating with good adherence and allows the production of a coating wherein the protein or proteins retain a very high level of nativity. Nativity may be measured using differential scanning calorimetry. An advantage of lyophilising is further the fact that the scaffold can be made without subjecting it to elevated temperatures (*e.g.* above 37°C). As such the method is also very suitable to incorporate pharmaceutically active substance, *e.g.* thermo-instable substances, in the scaffold, in particular in the coating.

Lyophilising is preferably performed at a temperature in the range of 0 to37°C, more preferably in the range of 15 to 25°C. The pressure during lyophilising is preferably in the range of 0.001 to 0.1 mbar, more preferably in the range of 0.01 to 0.08 mbar. Lyophilisation time can routinely be determined depending upon the other lyphilisation parameters. Typically a lyophilisation time of 16 to 48 hours will suffice. The temperature of the cooling spiral used is preferably between -10 and -80°C, more preferably between -30 and -60°C.

Optionally the collagen, the biological precursor of collagen or the combination thereof is cross-linked by a chemical treatment, a physical treatment or a combination thereof. By cross-linking the mechanical integrity and handling characteristics can be improved. Cross-linking can also be performed to decrease susceptibility of the protein towards degradation by proteolytic enzymes and the immunogenicity.

Cross-linking is for example conveniently performed with gluteraldehyde, formaldehyde, biocompatible bifunctional aldehydes, hexamethylene diisocysanate, carbodiimides, polyglycerol polyglyceryl ether, glyoxal and mixtures thereof. Good results have been achieved with 1-ethyl 3-3-dimethyl amino propyl carbodiimide (EDC).

Optionally N-hydroxysuccinimide (NHS) is used in combination with a cross-linker, *e.g.* EDC. The addition of NHS tends to increase cross-linking efficiency and the degree of cross-linking, leading to a slower degrading protein phase in the scaffold. This could be advantageous to adjust the rate of release of a biologically active substance incorporated therein or to modulate tissue response.

Cross-linking is preferably carried out using 10-200 mM, more preferably 30-100 mM EDC and an amount of NHS roughly corresponding to two thirds of the amount of EDC used, *e.g.* an amount of 50 mM EDC may be combined with an amount of 30 mM NHS. The pH of the cross-linking mixture is preferably between 4 and 8, more preferably around 5.5. The duration of the cross-linking reaction may be chosen between 0 and 24 hours, preferably between 2 and 4 hours.

The invention will now be further elucidated by the following, non-restrictive examples.

### Example 1. Coating of collagen to porous PEGT/PBT scaffolds

The biodegradable elastomeric copolymer poly(ethylene glycol) terephthalate (PEGT)/ poly(butylene terephthalate) (PBT) was used to prepare porous scaffolds. Scaffolds with the composition 4000PEGT55PBT45 (indicated as aPEGTbPBTc were a is Mw PEGT, b and c the wt.% PEGT and PBT respectively) were prepared by solvent casting.

The general collagen coating procedure is as follows. Native insoluble bovine tendon type I collagen (0.5 gram) (Sigma Chemical Co., St Louis, USA) is suspended in 50 ml 0.5 M acetic acid (pH 2.5) at 4°C for 16 h. After the addition of 50 ml demineralized water (4°C), the collagen is homogenized in a Waring Blendor (550 W) at room temperature for 2 min. Subsequently, the collagen is filtrated using a 90 µm nylon gauze filter (TG 100, Schleicher & Schuell, Dasel, Germany). PEGT/PBT scaffolds of 4 x 4 cm² (approximately 125 mg) are placed in plastic petri-dishes (Greiner, 8 cm in diameter), and incubated with 10 ml collagen dispersion (0.5% (w/v)) for 4 h at 20°C under gentle shaking using a orbital plate shaker. Subsequently, the collagen-impregnated scaffolds are transferred to new petri-dishes, frozen at - 80°C for 2 h, and lyophilized for 16 h at 0.050 mBar. This results in the presence of a whitish, soft, felt-like, and homogenous collagen coating.

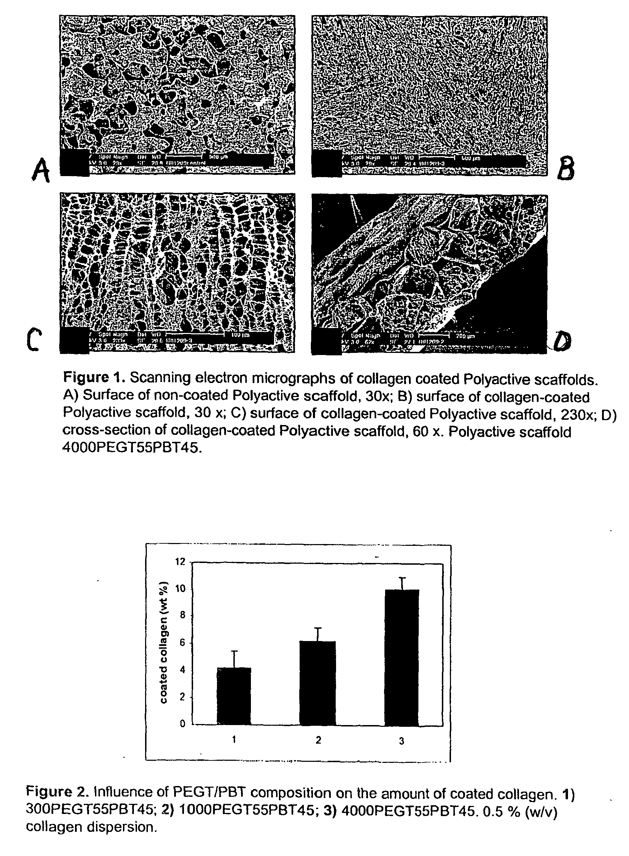

Scanning electron micrographs of the distribution and morphology of the collagen coating is shown in Figure 1. The coating entirely covers the porous PEGT/PBT scaffold. It consist of a collagenous meshwork with a fibrous, porous morphology (Fig. 1 B,C). The majority of the internal pores of the synthetic scaffold are also substantially filled with collagen (Fig. 1D). Fibrous meshworks predominate in these areas.

### Example 2. Modulation of collagen coating

Various parameters were evaluated to modulate the amount and morphology of the collagen coating. These include the composition of the PEGT/PBT scaffolds, the concentration and pH of the collagen dispersion, the impregnation time, the freezing temperature, and the influence of gasplasma pre-treatment. The feasibility of coating different scaffold designs, and the use of different coating techniques were assessed as well. The morphology of the collagen coating was analyzed with scanning electron microscopy (SEM). The amount of collagen coated was measured gravimetrically and expressed as ((dry weight collagen coated PEGT/PBT scaffold - dry weight PEGT/PBT scaffold)/(dry weight collagen coated PEGT/PBT scaffold)) * 100%.

### 2.1. Influence of PEGT/PBT composition on collagen coating

Collagen coatings were applied as described in Example 1. The influence of the PEGT/PBT composition on collagen coating was established by changing the molecular weight of the soft PEGT segment. Scaffold compositions varied from the hydrophobic 300PEGT55PBT45 to hydrophilic 4000PEGT55PBT45. As is shown in Figure 2, the amount of collagen coated on 300PEGT55PBT45 compositions is about 4 % (w/w), which increases to 10% (w/w) for the 4000PEGT55PBT45 scaffolds. The larger amount of collagen coated on these latter scaffolds is due to their ability to swell during incubation.

### 2.2. Influence of collagen concentration on collagen coating

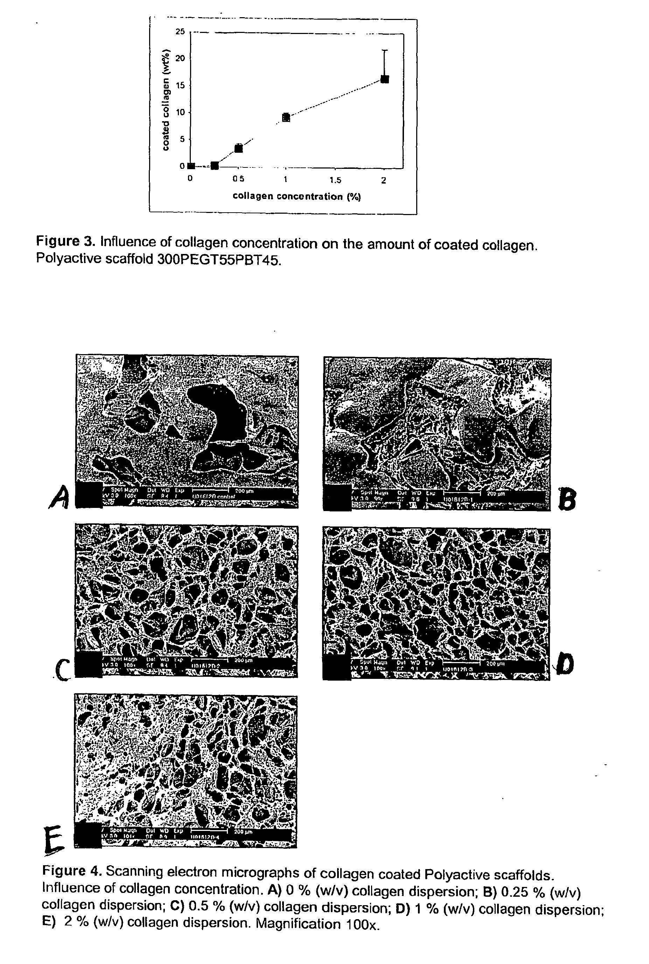

The influence of the collagen concentration on the amount of collagen coated is presented in Figure 3. In these experiments synthetic scaffolds with the composition 300PEGT55PBT45 were used. Coating was performed as described previously in Example 1. After incubation in a collagen dispersion of 0.25% (w/v), the amount of collagen coated is 0.25% (w/w), which considerably increases to approximately 16 % (w/w) after impregnation in a 2% (w/v) collagen dispersion.

Characteristic SEM pictures of collagen coated scaffolds are presented in Figure 4. At 0.25 % (w/v) collagen dispersion, the pores of the scaffold are filled with a fibrous collagen mesh, whereas at the outer sides the synthetic polymer structures are uncoated (Fig. 4B). At a collagen dispersion of 0.5% (w/v), a homogenous porous collagen meshwork with pore diameters ranging from approximately 50 to 200 µm is formed and completely covers the synthetic scaffold (Fig. 4C). A comparable coating morphology is obtained after incubation of the scaffold in a 1% (w/v) collagen dispersion (Fig. 4D). Using a 2% (w/v) collagen dispersion, porous collagen meshworks are still present, but dens, sheet-like collagen structures predominate (Fig. 4E).

### 2.3. Influence of pH on collagen coating

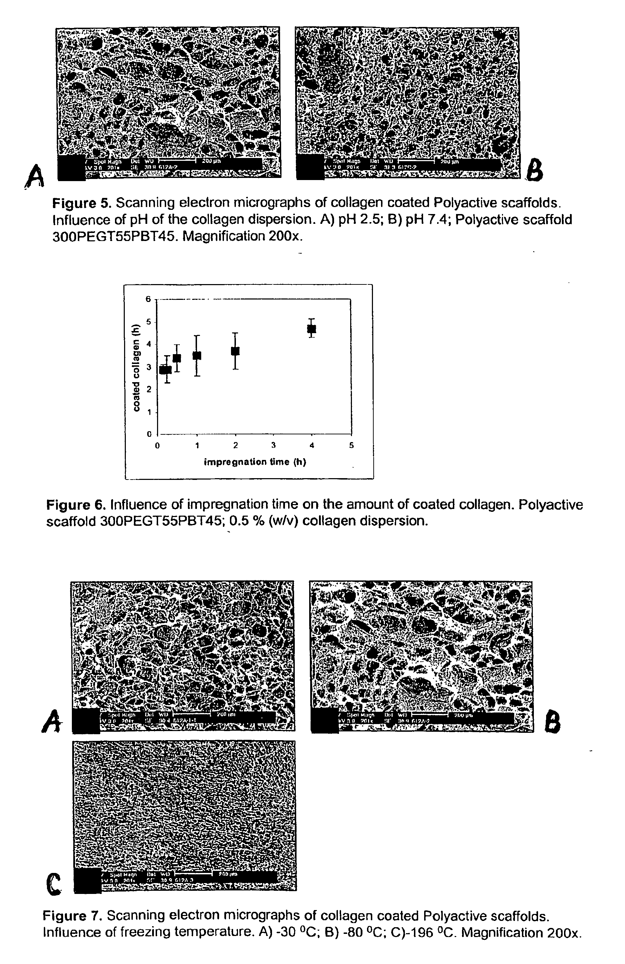

The influence of the pH of the collagen dispersion on the morphology of the collagen was also assessed. Increasing the pH of the dispersion from 2.5 to 7.4 results in the presence of a more dens and compact collagen coating (compare Fig. 5A and 5B). Small, uncoated PEGT/PBT domains are occasionally observed.

### 2.4. Influence of impregnation time on collagen coating

The influence of the impregnation time on the amount of collagen coated is shown in Figure 6. After 0.5 h, approximately 3% (w/w) collagen is coated to 300PEGT55PBT45 scaffolds, which increases to about 4.5% (w/w) after 4 h. The amount of coated collagen levels off after x h.

### 2.5. Influence of freezing temperature on collagen coating

Collagen coatings were applied as described using Polyactive 300PEGT55PBT45 scaffolds and a 0.5% (w/v) collagen dispersion. Collagen-impregnated scaffolds were frozen at -30, -80, and -196°C. Hybrid scaffolds were evaluated with SEM. A homogenous porous collagen meshwork is observed using a freezing temperature of -30°C (Fig. 7A). Occasionally, small sheet-like structures are observed. At -80°C, the collagen meshwork is more dens and compact compared to collagen coatings at -30°C, but the porous morphology is maintained (Fig 7B). At -196°C, a homogenous microporous meshwork with pores ranging from approximately 10-20 µm entirely coats the synthetic scaffold as is shown in Figure 7C.

### 2.6. Influence of gasplasma pre-treatment on collagen coating

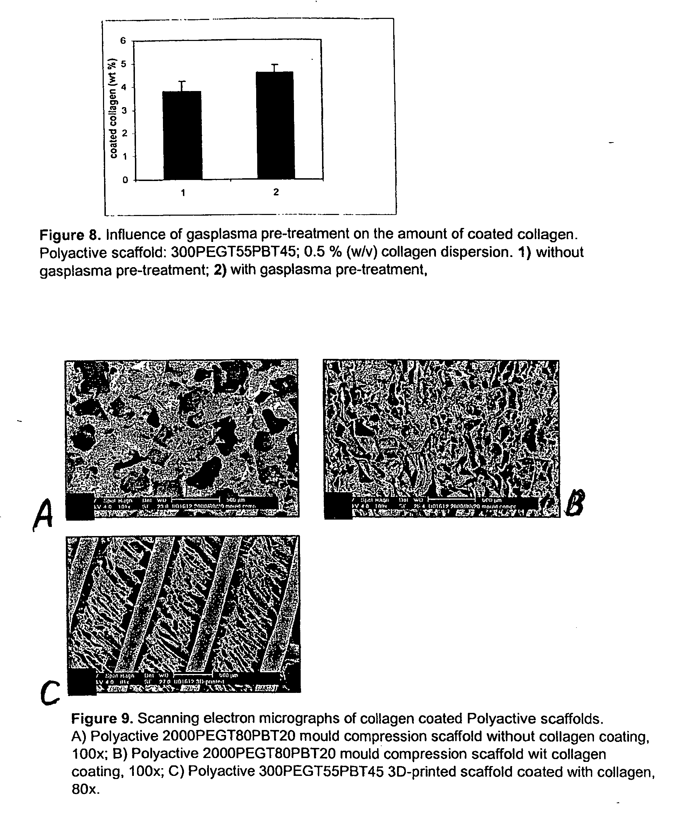

Porous 300PEGT55PBT45 scaffolds were gasplasma treated with argon for 10 min at 0.1-0.2 mBar using an Expanded Plasma Cleaner (PDC-002, 200 W, Harrick Scientific Corporation). Collagen coatings were applied as described in Example 1. The amounts of coated collagen, with and without gasplasma pre-treatment are presented in Figure 8 For non-gasplasma treated 300PEGT55PBT45 scaffolds the amount of coated collagen is 3.8 ± 0.4% (w/w) (mean ± SD, n=4), which increases to 4.6 ± 0.3% (w/w) (mean ± SD, n=4) for gasplasma treated compositions.

### 2.7. Feasibility of coating different scaffold designs

The feasibility of coating collagen on different scaffold designs was established. The designs included porous scaffolds prepared by mould compression techniques and 3D-printing. A collagen-coated mould compression scaffold is presented in Figure 9B. A fibrous and porous collagen meshwork completely covers the surface. 3D-printed PEGT/PBT scaffolds reveal highly oriented lattice-like laminar structures in between the polymer fibers of the printed scaffold (Fig. 9C).

### 2.8 Influence of coating technique on collagen coating morphology

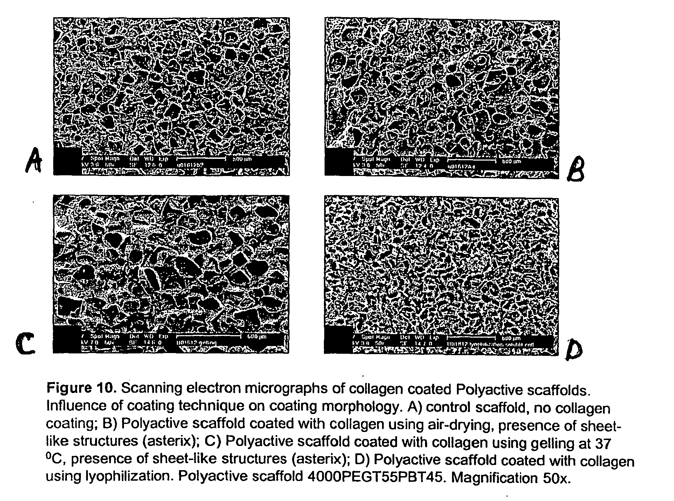

The lyophilization procedure was compared to collagen coating techniques known in the art. These include air-drying and gelling at 37°C of collagen impregnated synthetic scaffolds. Porous Polyactive scaffolds with a copolymer composition of 4000PEGT55PBT45 were impregnated for 4 h in 0.4% (w/v) acid soluble type I rat tail collagen (Sigma) at room temperature. Subsequently, collagen coatings were formed by either air-drying in a flow cabinet for 16 h at room temperature, gelling the collagen by incubation of scaffolds for 16 h at 37°C, or freezing the scaffolds at -80°C followed by lyophilization. Characteristic SEM pictures of the various collagen coatings are given in Figure 10. Both air-drying (Fig. 10B) and gelling techniques (Fig. 10C) resulted in the presence of sheet-like structures on the porous surface. By contrast, lyophilization revealed the typical porous collagen meshwork (Fig. 10D). All techniques resulted a comparable amount of coated collagen.

### Example 3. Bioabsorbability of collagen coatings evaluated in vitro

Resorbtion of the collagen coating from polymer/collagen hybrid composites was evaluated by incubation of these scaffolds in PBS and collagenase. Results are presented in Table 1.

### 3.1. Bioabsorbability of collagen coating at PBS incubation

Bioabsorbability of collagen coatings was analyzed by incubation of scaffolds of approximately 30 mg in PBS (pH 7.4) containing 0.01% (w/v) sodium azide at 37°C for time periods up to 16 days. Incubation medium was changed every two days. At designated time intervals, scaffolds were washed in demineralized water (2 times for 30 min) and lyophilized. Collagen coated 4000PEGT55PBT45 scaffolds, with and without gasplasma pre-treatment, and 4000PEGT55PBT45 scaffolds with a crosslinked collagen coating were included. The amount of resorbed collagen was determent gravimetrically and expressed as a percentage of the initial dry weight of the coated scaffolds.

Crosslinking of collagen coating was performed using the water soluble carbodiimide 1-ethyl-3-(3-dimethyl aminopropyl)carbodiimide (EDC) and N-hydroxysuccinimide (NHS). Collagen coated PEGT/PBT scaffolds were incubated in 50 mM 2-morpholinoethane sulfonic acid (pH 5.5) containing 50 mM EDC and 30 mM NHS. After reaction for 4 h at 20 °C, the hybrid composites were washed twice in 0.1 M Na₂HPO₄ (pH 9.1) for 1 h, 4 times in demineralized water for 30 min, and lyophilized. After 16 days, the collagen coating of Polyactive/collagen composites was resorbed by 35% (Table 1). The use of gasplasma pre-treatments decreased the amount of resorbed collagen to 23%. Resorbtion of collagen was additionally decreased to 15% by EDC and NHS crosslinking of the collagen coating.

### 3.2. Bioabsorbability of collagen coating at collagenase treatment

Bioabsorbability of coated collagen, with and without crosslinking, was also assessed by proteolytic digestion with collagenase. Crosslinking with EDC and NHS was performed as described in Example 3.1. Collagen coated scaffolds of approximately 30 mg in dry weight were incubated in 10 ml 0.1 M Tris-HCl (pH 7.4) containing 0.05 M CaCl₂ and 100 U/ml bacterial collagenase (Clostridium Histolyticum) at 37°C. After 40 minutes, scaffolds were washed in 0.1 M EDTA (2 times for 30 min), demineralized water, and lyophilized. Coated collagen without gasplasma pre-treatment were completely degraded at collagenase treatment. By contrast, crosslinked collagen coatings were only degraded by 36% (Table 1).

### Example 4. Collagen coated PEGT/PBT scaffold as a carrier for the transfer of keratinocytes

In this experiment, porous 4000PEGT55PBT45 scaffolds, with and without a collagen coating, were used as a carrier for the transfer of autologous undifferentiated keratinocytes in porcine full-thickness wounds. The use of such a carrier may stimulate wound closure and provide a suitable alternative to available techniques like meshed split thickness skin grafts or cultured epithelial autografts for the treatment of burns or chronic wounds.

Protocols were approved by the Animal Welfare Committee at the Animal Institute of the Academic Medical Centre in Amsterdam. Two female Yorkshire pigs (30 ± 5 kg) were included in the study. Porcine keratinocytes were isolated from split skin grafts of donor sites on the back and lower back of the pigs, and cultured in Optimem^{®} based medium. One day before the operation, keratinocytes were seeded in the scaffolds at a density of 500.000 cells/cm². General anesthesia was performed by intramuscular administration of Stresnil^{®}, and Nimatec^{®}. During operation a combination of intramuscular administered Sufenta^{®}, and inhalation gasses O₂/N₂O (ratio 1:1.5) and Isoflurane were used. The skin area was cleaned with a mild detergent solution, shaved using common razor blades, cleaned again, and disinfected using a 2% Poviodine solution. Full-thickness skin wounds of 3 x 3 cm² were created on the paravertebral region on both flanks by excision down to the adipose tissue. PEGT/PBT scaffolds (3 x 3 cm²), with and without collagen coating, and with and without keratinocytes were applied to the wounds. Cell seeded scaffolds were applied in an up-side down fashion, i.e. the cell seeded side of the scaffold being in direct contact with the wound bed. Scaffolds were fixed to the wound using surgical staples. Wounds were bandaged with Allyvin (Smith & Nephew Medical, England) and elastic adhesive tape. Finally, wounds were covered with hydrophilic gauzes for protection against mechanical trauma, elastic adhesive tape, and elastic stocking. At day 4, 7, 11 and 21, wounds were inspected macroscopically and biopsies were taken for histological analyses.

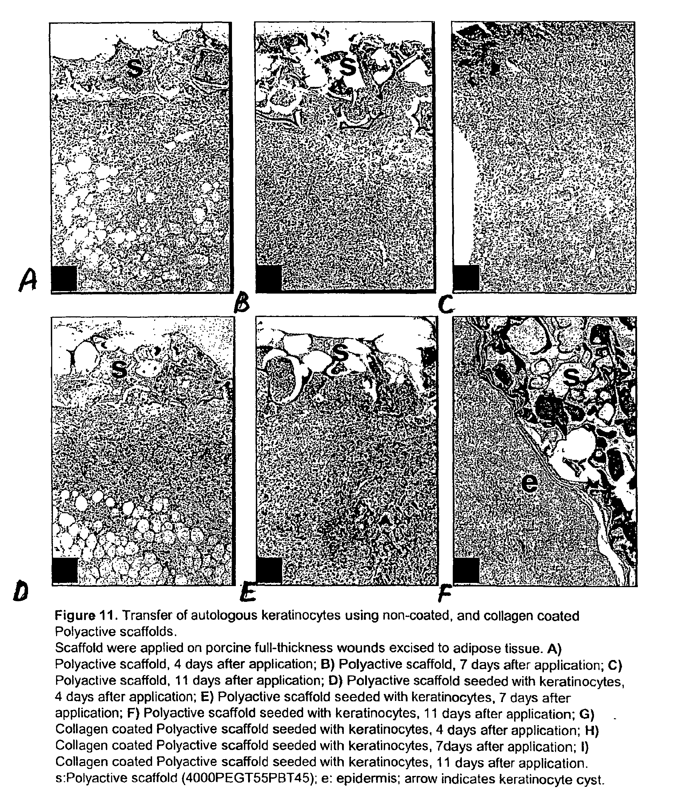

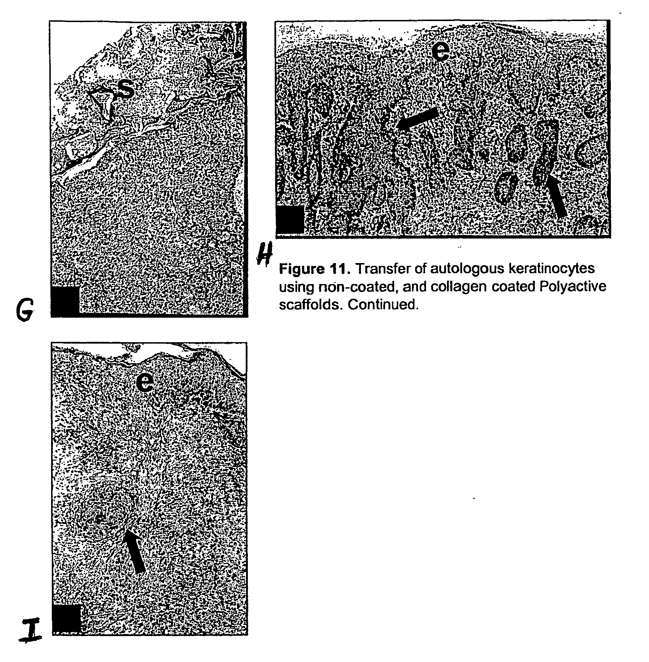

Histological section taken from the middle of the wounds are given in Figure 11. At day 4 and 7, the 4000PEGT55PBT45 scaffolds, with and without collagen coating, revealed only little granulation tissue ingrowth, the scaffold being mainly located on top of the wound and in the crust (Fig. 11A,B). At day 11, wounds are not closed and small scaffold fragments can be observed on top of the granulation tissue (Fig. 11C). By contrast, 4000PEGT55PBT45 scaffolds, seeded with keratinocytes, revealed a complete epidermis after 11 days, indicating transfer of keratinocytes (Fig. 11F). Transfer was substantially stimulated in the presence of a collagen coating. Collagen coated 4000PEGT55PBT45 scaffolds, seeded with keratinocytes, showed epidermis formation and the presence keratinocyte cysts as early as 7 days after application (Fig. 11H). After 11 days, the size and number of these cysts have decreased, indicating normalization of the newly formed epidermis (Fig. 11I). No differences in wound contraction were observed between the different treatment modalities. Results demonstrate that PA/collagen hybrid scaffolds are suitable carriers for the transfer of keratinocytes to promote wound healing.

## Claims

1. A method for manufacturing a biocompatible scaffold, wherein a matrix comprising a copolymer of a polyalkylene glycol terephthalate and an aromatic polyester is provided; the matrix is contacted with an aqueous solution or dispersion comprising at least one protein selected from the group consisting of collagens and biological precursors and functional analogues thereof; and wherein the matrix is subsequently dried by lyophilising to form the scaffold.

2. A method according to claim 1, wherein the contacting of matrix and aqueous mixture is carried out while shaking or centrifuging the same.

3. A method according to claim 1 or 2, wherein the collagen, the biological precursor of collagen or the combination thereof is cross-linked by a chemical treatment, a physical treatment or a combination thereof.

4. A method according to any of the preceding claims, wherein the collagen is selected from the group consisting of human collagens, animal derived collagens, recombinant collagens, and combinations thereof.

5. A method according to any of the preceding claims, wherein the collagen precursor is selected from the group consisting of procollagens.

6. A method according to any of the preceding claims, wherein said at least one protein is non-covalently conjugated to the copolymer.

7. A method according to any of the preceding claims, wherein the polyalkylene glycol terephthalate in the copolymer is selected from the group consisting of copolymers of polyethylene glycol terephthalate, polypropylene glycol terephthalate, polybutylene glycol terephthalate and combinations thereof and wherein the aromatic polyester is selected from the group consisting of polyethylene terephthalate, polypropylene terephthalate, polybutylene terephthalate and combinations thereof.

8. A method according to any of the preceding claims, wherein the aqueous mixture comprises a pharmaceutically active substance.

9. A method according to claim 8, wherein the pharmaceutically active substance is selected from the group consisting of hormones, growth factors, antibiotics, antimicrobial peptides, anti-inflammatory agents, chemotactic agents, anti-fungal agents and combinations thereof.

10. Biocompatible scaffold obtainable by a method according to any of the preceding claims.

11. A scaffold according to claim 10, which scaffold is formed of a matrix comprising the copolymer of a polyalkylene glycol terephthalate and an aromatic polyester, which matrix is provided with a coating comprising said at least one protein.

12. A scaffold according to claim 10 or 11, wherein the scaffold has interconnected pores, oriented fibres, laminar structures or a combination thereof.

13. A scaffold according to claim 12, wherein at least the majority of the pores in the porous scaffold has an average diameter in the range of 10-1,000 µm.

14. A scaffold according to any of claims 10-13 comprising living cells, preferably autologous cells.

15. A drug-delivering device, a dermal substitute or a cartilage substitute comprising a scaffold according to any of the claims 10-14.

16. Use of a scaffold according to any of the claims 10-14 in the manufacture of a drug-delivering device, a dermal substitute or a cartilage substitute.

## Patentansprüche

1. Ein Verfahren zum Herstellen eines biologisch verträglichen Gerüstes, worin eine Matrix, umfassend ein Polymer von einem Polyalkylenglykolterephthalat und einem aromatischen Polyester bereitgestellt wird; die Matrix in Kontakt gebracht wird mit einer wässrigen Lösung oder Dispersion, umfassend mindestens ein Protein, ausgewählt aus der Gruppe, bestehend aus Kollagenen und biologischen Vorläufern und funktionellen Analoga davon; und worin die Matrix anschließend durch Lyophilisierung getrocknet wird, um das Gerüst zu bilden.

2. Ein Verfahren nach Anspruch 1, worin das Inkontaktbringen der Matrix und der wässrigen Mischung durchgeführt wird, während dieselben geschüttelt oder zentrifugiert werden,

3. Ein Verfahren nach Anspruch 1 oder 2, worin das Kollagen, der biologische Vorläufer von Kollagen oder die Kombination davon durch eine chemische Behandlung, eine physikalische Behandlung oder eine Kombination davon vernetzt wird.

4. Ein Verfahren nach einem der vorherigen Ansprüche, worin das Kollagen ausgewählt ist aus der Gruppe, bestehend aus menschlichen Kollagenen, Kollagen, welches von Tieren abgeleitet wird, rekombinanten Kollagenen und Kombinationen davon.

5. Ein Verfahren nach einem der vorherigen Ansprüche, worin der Kollagenvorläufer ausgewählt ist aus der Gruppe, bestehend aus Pro-Kollagenen.

6. Ein Verfahren nach einem der vorherigen Ansprüche, worin das mindestens eine Protein nicht kovalent konjugiert zu dem Copolymer ist.

7. Ein Verfahren nach einem der vorherigen Ansprüche, worin das Polyalkylenglykolterephthalat in dem Copolymer ausgewählt ist aus der Gruppe, bestehend aus Copolymeren von Polyethylenglykolterophthalat, Polypropylenglykolterephthalat, Polybutylenglykolterephthalat und Kombinationen davon, und worin der aromatische Polyester ausgewählt ist aus der Gruppe, bestehend aus Polyethylenterephthalat, Polypropylenterephthalat, Polybutylenterephthalat und Kombinationen davon,

8. Ein Verfahren nach einem der vorherigen Ansprüche, worin die wässrige Mischung einen pharmazeutisch aktiven Wirkstoff umfasst.

9. Ein Verfahren nach Anspruch 8, worin der pharmazeutisch aktive Wirkstoff ausgewählt ist aus der Gruppe, bestehend aus Hormonen, Wachstumsfaktoren, Antibiotika, antimikrobiotisch wirkenden Peptiden, entzündungshernmendem Mittel, chemotaktischen Mitteln, antimykotischen Mitteln und Kombinationen davon,

10. Biologisch verträgliches Gerüst, erhältlich nach einem Verfahren gemäß einem der vorherigen Ansprüche.

11. Ein Gerüst nach Anspruch 10, worin das Gerüst aus einer Matrix, umfassend das Copolymer eines Polyalkylenglykolterephthalats und eines aromatischen Polyesters, gebildet wird, wobei die Matrix mit einer Beschichtung, umfassend zumindest ein Protein, ausgestattet ist.

12. Ein Gerüst nach Anspruch 10 oder 11, worin das Gerüst miteinander verbundene Poren, orientierte Fasern, laminare Strukturen oder eine Kombination davon aufweist.

13. Ein Gerüst nach Anspruch 12, worin zumindest die Mehrzahl der Poren In dem porösen Gerüst einen mittleren Durchmesser in dem Bereich von 10 bis 1000 µm aufweist.

14. Ein Gerüst nach einem der Ansprüche 10 bis 13, umfassend lebende Zellen, vorzugsweise autogenetische Zellen.

15. Eine Zuführvorrichtung für Arzneimittel, ein Hautsubstitut oder ein Knorpelsubstitut, umfassend ein Gerüst gemäß einem der Ansprüche 10 bis 14.

16. Verwendung eines Gerüstes gemäß einem der Ansprüche 10 bis 1 4 in der Herstellung einer Vorrichtung zur Arzneimittelzuführung, eines dermalen Substituts oder eines Knorpelsubstituts.

## Revendications

1. Procédé de fabrication d'un échafaudage biocompatible, dans lequel on fournit une matrice comprenant un copolymère d'un poly(téréphtalate d'alkylèneglycol) et d'un polyester aromatique; on met la matrice en contact avec une solution ou une dispersion aqueuse comprenant au moins une protéine choisie dans le groupe constitué par des collagènes et leurs précurseurs biologiques et leurs analogues fonctionnels; et on sèche ensuite la matrice par lyophilisation pour former l'échafaudage.

2. Procédé selon la revendication 1, dans lequel on effectue la mise en contact de la matrice et du mélange aqueux tout en les agitant ou en les centrifugeant.

3. Procédé selon la revendication 1 ou 2, dans lequel le collagène, le précurseur biologique du collagène ou leur combinaison sont réticulés par traitement chimique, traitement physique ou une de leurs combinaisons.

4. Procédé selon l'une quelconque des revendications précédentes, dans lequel le collagène est choisi dans le groupe constitué par des collagènes humains, des collagènes provenant d'animaux, des collagènes recombinés et leurs combinaisons.

5. Procédé selon l'une quelconque des revendications précédentes, dans lequel le précurseur de collagène est choisi dans le groupe constitué par des procollagènes.

6. Procédé selon l'une quelconque des revendications précédentes, dans lequel ladite au moins une protéine est conjuguée de manière non covalente au copolymère.

7. Procédé selon l'une quelconque des revendications précédentes, dans lequel le poly(téréphtalate d'alkylèneglycol) dans le copolymère est choisi dans le groupe constitué par des copolymères de poly(téréphtalate d'éthylèneglycol), de poly(téréphtalate de propylèneglycol), de poly(téréphtalate de butylèneglycol) et leurs combinaisons, et dans lequel le polyester aromatique est choisi dans le groupe constitué par du poly(téréphtalate d'éthylène), du poly(téréphtalate de propylène), du poly(téréphtalate de butylène) et leurs combinaisons.

8. Procédé selon l'une quelconque des revendications précédentes, dans lequel le mélange aqueux comprend une substance pharmaceutiquement active.

9. Procédé selon la revendication 8, dans lequel la substance pharmaceutiquement active est choisie dans le groupe constitué par des hormones, des facteurs de croissance, des antibiotiques, des peptides antimicrobiens, des agents anti-inflammatoires, des agents chimiotactiques, des agents antifongiques et leurs combinaisons.

10. Échafaudage biocompatible pouvant être obtenu un procédé selon l'une quelconque des revendications précédentes.

11. Échafaudage selon la revendication 10, lequel échafaudage est formé d'une matrice comprenant le copolymère d'un poly(téréphtalate d'alkylèneglycol) et d'un polyester aromatique, laquelle matrice est pourvue d'un revêtement comprenant ladite au moins une protéine.

12. Échafaudage selon la revendication 10 ou 11, l'échafaudage ayant des pores interconnectés, des fibres orientées, des structures laminaires ou une de leurs combinaisons.

13. Échafaudage selon la revendication 12, dans lequel au moins la majeure partie des pores de l'échafaudage poreux a un diamètre moyen dans le domaine de 10-1 000 µm.

14. Échafaudage selon l'une quelconque des revendications 10-13, comprenant des cellules vivantes, de préférence des cellules autologues.

15. Dispositif de délivrance de médicaments, substitut dermique ou substitut de cartilage comprenant un échafaudage selon l'une quelconque des revendications 10-14.

16. Utilisation d'un échafaudage selon l'une quelconque des revendications 10-14 dans la fabrication d'un dispositif de délivrance de médicaments, d'un substitut dermique ou d'un substitut de cartilage.
